# EUROPEAN PATENT SPECIFICATION

(11) **EP 1 791 070 B1**
(45) Date of publication and mention of the grant of the patent: **30.05.2012**
(21) Application number: 06124638.5
(22) Date of filing: 23.11.2006
(51) Int. Cl.: G06F 19/00, A61B 17/00, A61B 19/00

(54) **Systems for facilitating surgical procedures**
Systeme zur Erleichterung chirurgischer Verfahren
Systèmes destinés à faciliter les procédures chirurgicales

(30) Priority: 23.11.2005 US 286549
(43) Date of publication of application: 30.05.2007
(73) Proprietor: GENERAL ELECTRIC COMPANY, Schenectady, NY 12345 (US)
(72) Inventor: Mahesh, Prakash, Hoffman Estates, IL 60192 (US); Morita, Mark M., Arlington Heights, IL 60004 (US)
(74) Representative: Illingworth-Law, William Illingworth

(56) References cited:
- WO-A2-2004/001569
- US-A1- 2004 034 282
- US-A1- 2005 159 759

## Description

### BACKGROUND OF THE INVENTION

Embodiments of the present application relate generally to facilitating surgical procedures. Particularly, certain embodiments relate to providing systems for following a pre-operative surgical plan to efficiently performing surgery.

Surgery may have associated risks. It may, therefore, be desirable to both clinicians and patients to reduce both the magnitude and probability of any such surgical risks. One way to reduce risk may be to improve pre-operative planning. Improved pre-operative planning may reduce the time for a procedure, and the number of invasive actions which may be performed, for example. Additionally, improved pre-operative planning may decrease any risk of interference with healthy, sensitive tissues and/or organs (e.g. blood vessels or nerves) in the potential path of surgical instruments. Furthermore, certain techniques for improving radiological image quality, such as administration of a contrast agent to a patient, may not be employed during surgery. Therefore, a surgeon or other clinician may employ such techniques during a planning stage to better ascertain the nature of a patient's anatomy.

Pre-operative planning, however may be time consuming. Furthermore, clinicians may lack tools that provide readily obtainable information for pre-operative planning. While a clinician may have access to a radiological image of the patient, the image may require significant analysis by clinicians. Manual analysis of radiological images may be time consuming and expensive. Additionally, manual analysis may not result in electronically available plans capable of real-time interaction, such as during surgery. Manual plans may not be electronically monitored during a surgical procedure. Furthermore, manual analysis may be difficult, because three dimensional information is generally being shown to the clinician on a display which is only two dimensional.

One particular type of procedure in need of improved pre-operative planning may be called ablation (e.g. thermal ablation or cryoablation). Ablation (e.g. thermal ablation or cryoablation) may be any surgical excision of tissue, such as a tumor, or a portion thereof. One form of ablation (e.g. thermal ablation or cryoablation) involves insertion of an ablation tool into a tissue to be removed. The ablation tool tip may then achieve a high temperature for a particular duration, thereby causing the tissue to be to be killed. In thermal ablations, the tissue may be essentially boiled whereas in cryoablation, the tissue may be frozen and killed. Various tool tips may be available, each tip capable of removing a different amount of tissue under various circumstances. It may be difficult for clinicians to calculate the volumetric effects of various ablation tools during pre-operative planning.

The state of the art is represented by WO-A-2004/001569 and aknowledged in the preamble of claim 1.

Thus, there is a need for methods and systems that reduce risks associated with surgical procedures. There is a need for methods and systems that automatically provide pre-operative plans for later use in a surgical setting. Additionally, there is a need for methods and systems that assist clinicians in following pre-operative plans during a surgery.

### BRIEF SUMMARY OF THE INVENTION

The system according to the invention is defined by claim 1.

A method for facilitating surgery can be carried out with the system of the present invention, the method including: tracking a position of at least a portion of a surgical implement in a volume of interest; recognizing a surgical plan corresponding to at least a portion of the volume of interest; and providing feedback based on a correspondence between the position of the at least a portion of the surgical implement and the surgical plan, wherein said surgical plan is determined by a method comprising: providing for display a data set including a representation of a volume of interest, providing an interactive tool representative of said at least a portion of a surgical implement for use in conjunction with the data set, allowing an interaction between a user directing said interactive tool and said data set, forming a prediction based on said interaction, recording said interaction of the user and storing said recorded interaction of the user as a surgical plan. In an embodiment, the feedback is provided in real-time. In an embodiment, the feedback includes at least one of: haptic feedback, thermal feedback, optical feedback, and auditory feedback. In an embodiment, the surgical plan includes a previously generated radiological image. In an embodiment, the surgical plan includes at least one trajectory for the at least a portion of the surgical implement. In an embodiment, the surgical plan includes at least one ablation. In an embodiment, the method further includes displaying a real-time radiological image of at least a portion of the volume of interest, wherein the real-time radiological image corresponds to the surgical plan. In an embodiment, the real-time radiological image includes an ultrasound image. In an embodiment, the feedback is provided to a clinician.

In an embodiment of the inventive system, the surgical plan includes at least one trajectory and at least one position. In an embodiment of the inventive system, the feedback response includes at least one of: haptic feedback, thermal feedback, optical feedback, and auditory feedback. In an embodiment of the inventive system, the feedback response is provided in real-time. In an embodiment of the inventive system, the system further includes a displayable output generated by the application, wherein the displayable output includes a real-time radiological image of at least a portion of the patient, wherein the real-time radiological image corresponds to the surgical plan. In an embodiment, the displayable output further includes a previously generated radiological image integrated with the real-time radiological image. In an embodiment, the displayable output further includes the position of the at least a portion of the surgical implement. In an embodiment, the surgical plan further includes a segmentation. In an embodiment, the surgical implement includes an ablation tool. In an embodiment, the feedback response is provided to a clinician.

Certain embodiments provide a computer-readable storage medium including a set of instructions for a computer, the set of instructions including: a tracking routine for tracking a position of at least a portion of a surgical implement in a volume of interest; a recognition routine for recognizing a surgical plan corresponding to at least a portion of the volume of interest; and a feedback routine for providing feedback based on a correspondence between the position of the at least a portion of the surgical implement and the surgical plan. In an embodiment, the feedback is provided in real-time. In an embodiment, the feedback includes at least one of: haptic feedback, thermal feedback, optical feedback, and auditory feedback. In an embodiment, the surgical plan includes a previously generated radiological image. In an embodiment, the surgical plan includes at least one trajectory for the at least a portion of the surgical implement. In an embodiment, the surgical plan includes at least one ablation. In an embodiment, the set of instructions further includes a display routine for displaying a real-time radiological image of at least a portion of the volume of interest, wherein the real-time radiological image corresponds to the surgical plan. In an embodiment, the real-time radiological image includes an ultrasound image. In an embodiment, the feedback is provided to a clinician.

### BRIEF DESCRIPTION OF SEVERAL VIEWS OF THE DRAWINGS

FIG. 1 shows a system for performing surgical planning, in accordance with an embodiment of the present invention.
FIG. 2 shows a method for performing surgical planning, iwhich can be carried out with the system of the present invention.
FIG. 3 shows an example of segmentation, in accordance with an embodiment of the present invention.
FIG. 4 shows an example of an application display displaying data and an interactive tool, in accordance with an embodiment of the present application.
FIG. 5 shows an example of prediction forming, in accordance with an embodiment of the present invention.
FIG. 6 shows a method for performing automated surgical planning.
FIG. 7 shows a system for facilitating surgery, in accordance with an embodiment of the present invention.
FIG. 8 shows a flowchart for a method for facilitating surgery.
FIG. 9 shows an example of a combination display including a surgical plan and a three-dimensional real-time image of a volume of interest, in accordance with an embodiment of the present invention.

The foregoing summary, as well as the following detailed description of certain embodiments of the present application, will be better understood when read in conjunction with the appended drawings. For the purpose of illustrating the invention, certain embodiments are shown in the drawings. It should be understood, however, that the present invention is not limited to the arrangements and instrumentality shown in the attached drawings. Further, some figures may be representations of the type of display and/or output associated with systems of the present invention, in accordance with one or more embodiments.

### DETAILED DESCRIPTION OF THE INVENTION

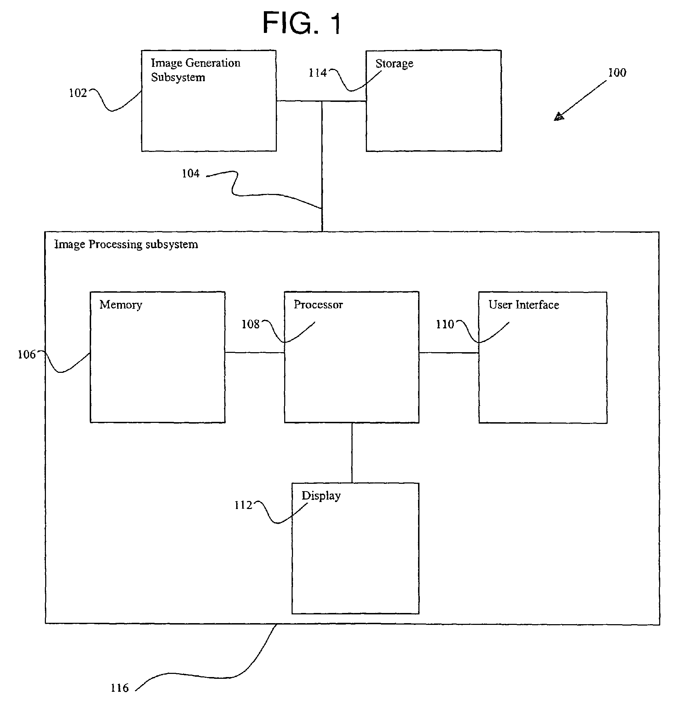

FIG. 1 shows a system 100 for performing surgical planning, in accordance with an embodiment of the present invention. A system 100 may include an image generation subsystem 102 communicatively linked to an image processing subsystem 116 and/or a storage 114 through one or more communications links 104. One or more components, such as storage 114, may be omitted from system 100, for example. One or more components may be integrated in various forms, or may be distributed across a plurality of components in various forms, for example.

An image generation subsystem 102 may be any radiological system capable of generating two-dimensional, three-dimensional, and/or four-dimensional data corresponding to a volume of interest of a patient. A volume of interest of a patient may include tissue, organs, fluids, pathologies (e.g. tumors, abscesses, cysts, etc.), and/or the like. Some types of image processing subsystems 102 include computed tomography (CT), magnetic resonance imaging (MRI), x-ray, positron emission tomography (PET), tomosynthesis, and/or the like, for example. An imaging modality, such as CT, may be enhanced through a contrast agent administered to a patient, for example. An image generation subsystem 102 may generate one or more data sets corresponding to an image which may be communicated over a communications link 104 to a storage 114 and/or an image processing subsystem 116.

A storage 114 may be capable of storing set(s) of data generated by the image generation subsystem 102. The storage 114 may be, for example, a digital storage, such as a PACS storage, an optical medium storage, a magnetic medium storage, a solid-state storage, a long-term storage, a short-term storage, and/or the like. The storage 114 may be integrated with image generation subsystem 102 or image processing subsystem 116, for example. The storage 114 may be locally or remotely located, for example. The storage 114 may be persistent or transient, for example.

An image processing subsystem 116 may further include a memory 106, a processor 108, a user interface, 110 and/or a display 112. The various components of an image processing subsystem 116 may be communicatively linked. Some of the components may be integrated, such as, for example processor 108 and memory 106. An image processing subsystem 116 may receive data corresponding to a volume of interest of a patient. Data may be stored in memory 106, for example. An image processing subsystem 116 may include a computer, a PACS workstation, an Advantage® workstation, and/or the like, for example.

A memory 106 may be a computer-readable memory, for example, such as a hard disk, floppy disk, CD, CD-ROM, DVD, compact storage, flash memory, random access memory, read-only memory, electrically erasable and programmable read-only memory and/or other memory. The memory 106 may include more than one memory for example. The memory 106 may be able to store data temporarily or permanently, for example. The memory 106 may be capable or storing a set of instructions readable by processor 108, for example. The memory 106 may also be capable of storing data generated by image generation subsystem 102, for example. The memory 106 may also be capable of storing data generated by processor 108, for example.

A processor 108 may be a central processing unit, a microprocessor, a microcontroller, and/or the like. The processor 108 may include more than one processors, for example. The processor 108 may be an integrated component, or may be distributed across various locations, for example. The processor 108 may be capable of executing an application, for example. The processor 108 may be capable of executing methods, such as method 200, in accordance with the present invention, for example. The processor 108 may be capable of receiving input information from a user interface 110, and generating output displayable by a display 112, for example.

A user interface 110 may include any device(s) capable of communicating information from a user to an image processing subsystem 116, for example. The user interface 110 may include a mouse, keyboard, and/or any other device capable of receiving a user directive. For example, the user interface 110 may include voice recognition, motion tracking, and/or eye tracking features, for example. The user interface 110 may be integrated into other components, such as display 112, for example. As an example, the user interface 110 may include a touch responsive display 112, for example. Through user interface 110, a user may be capable of interacting with an application executing on processor 108, for example. Through user interface 110, a user may be capable of interacting with a data set storable in memory 106, for example. Through user interface 110, a user may be capable of interacting with an image displayable on display 112, for example.

A display 112 may be any device capable of communicating visual information to a user. For example, the display 112 may include a cathode ray tube, a liquid crystal diode display, a light emitting diode display, a projector and/or the like. The display 112 may be capable of displaying radiological images and data generated by image processing subsystem 116, for example. The display may be two-dimensional, but may be capable of indicating three-dimensional information through shading, coloring, and/or the like.

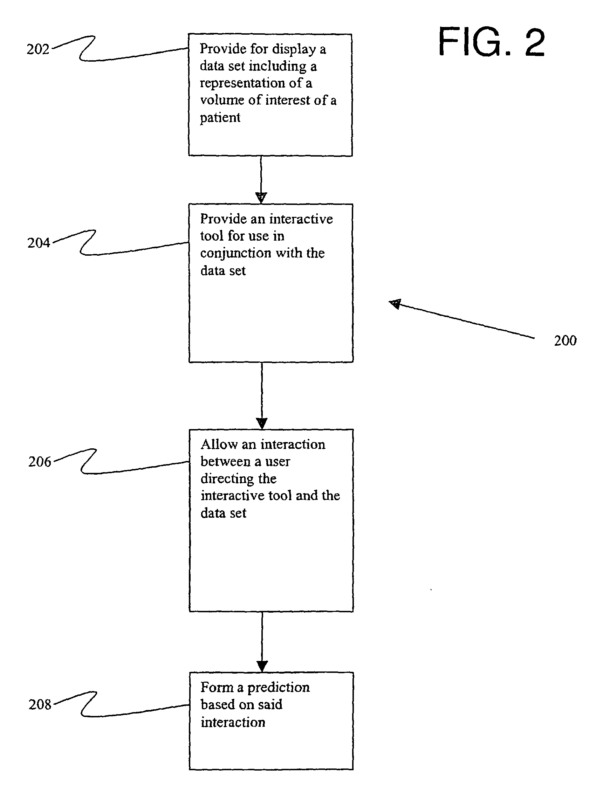

FIG. 2 shows a flowchart of a method 200 for performing surgical planning. The steps of method 200 may be performed in an alternate order as shown, for example. At least some of the steps of method 200 may be performed simultaneously or substantially simultaneously, for example. Furthermore, some steps of method 200 may be omitted, for example. The steps of method may be performed by a computer and/or other processor (such as processor 108 in FIG. 1) executing a set of instructions on a computer-readable medium, for example. Further, some steps of method 200 may be interchanged with similar steps in method 600, described below, and vice versa.

At step 202, data including a representation of a volume of interest of a patient is provided for display. For example, data may be provided for display on a display (e.g., display 112). For example, data may be generated by radiological imaging (e.g., image generation subsystem 102), and may include information representative of a volume of interest in a patient. Data may contain two, three, and/or four dimensional information, for example. Data may include one or more views of a volume of interest, such as axial, coronal, sagittal, and/or oblique views, for example. Data may be helpful to clinicians for planning and/or visualizing surgical procedures in two, three, and/or four dimensions, for example. For example, data may be helpful to an interventional clinician, such as an interventional radiologist, for planning interventional procedures.

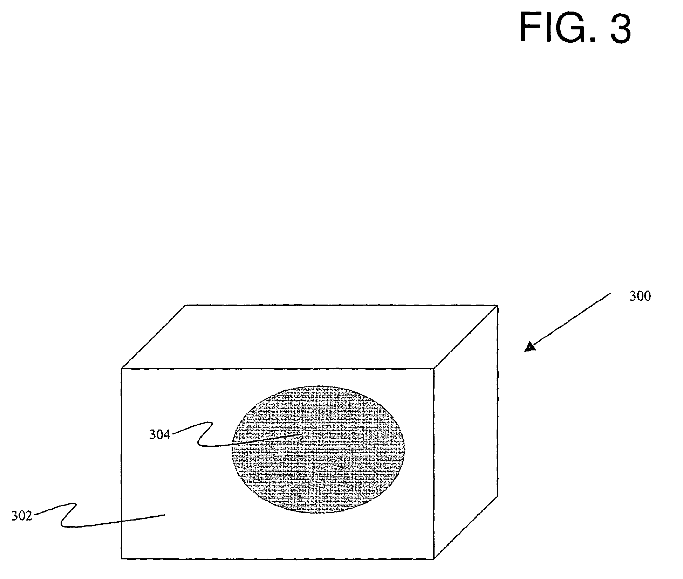

Turning for a moment to FIG. 3, an example of image 300 including segmentation is shown in accordance with an embodiment of the present invention. Data may include data from a radiological imaging system, and additional data, for example. In an embodiment, data includes segmentation 304 of biological structure represented in a radiological image 300. Segmentation 304 may include shapes or forms indicative of various biological structure, for example. For example, segmentation 304 may include an outlined form of a pathology, such as a tumor. Segmentation 304 may include forms indicative of organs and other tissues, such as blood vessels and/or nerves, for example.

Radiological image data 300 may contain a patient's anatomy, including a portion suitable for segmentation 304. Further, a segmentation 304 is shown within the volume of interest 302. The segmentation 304 may be generated by an application running on a processor, such as processor 108 shown in FIG. 1, for example. The segmentation 304 may represent forms of various biological structure, such as organs, tissues, and/or pathologies (e.g. tumors, cysts, etc.), for example. The process of segmentation 304 may be facilitated during image generation by administering an image enhancing agent to a patient, such as a contrast agent, for example.

Segmentation 304 may be formed based on varying intensity properties of pixels and/or voxels, for example. Various tissue, fluid, and/or organ types may be identified based on intensity properties of pixels and/or voxels, for example. A tissue type, such as bone, may cause pixels and/or voxels to have intensity properties within a range associated with bone, for example. A different tissue type, such as nerves, may cause pixels and/or voxels to have intensity properties within a range associated with nerves, for example. Various techniques may be employed to alter intensity properties associated with various anatomy, such as administering a contrast agent to a patient before imaging, for example. A contrast agent may be useful for altering intensity properties such that the intensity properties of various anatomy may be easier to differentiate. In other words, based on associated intensity properties, it may be easier to differentiate various anatomy in an image of a patient with a contrast agent than an image of a patient without a contrast agent, for example.

Based on expected intensity properties associated with pixels and/or voxels, it may be possible to filter various portions of an image. For example, if certain anatomy portions are not to be segmented, such portions may be filtered. For example, musculature, bone, blood vessels, nerves, and/or the like may be filtered, leaving behind a pathology, such as a tumor, for example. Alternatively, anatomy portions may be selected for segmentation based on associated intensity properties, for example. Blood vessels, for example, may be selected for segmentation based on associated intensity properties.

Once selected, a portion of anatomy may be segmented, for example. Various techniques may be used for segmentation, such as edge detection, for example, to form a segmentation 304. Segmentation 304 may be performed in two, three, and/or four dimensions, for example. The segmentation 304 may be two, three, and/or four dimensional, for example. Further processing and interaction may be performed with segmentation 304, for example. The segmentation 304 may be storable in memory (such as memory 106, for example) as a separate data set, or integrated and/or in association with the radiological image data, for example.

Turning back to FIG. 2, at step 204, an interactive tool is provided for use in conjunction with the data set (e.g. radiological image data 302 and segmentation 304). An interactive tool may be one or more tools with which a user may interact. For example, a user through a user interface (such as user interface 110) may select an interactive tool.

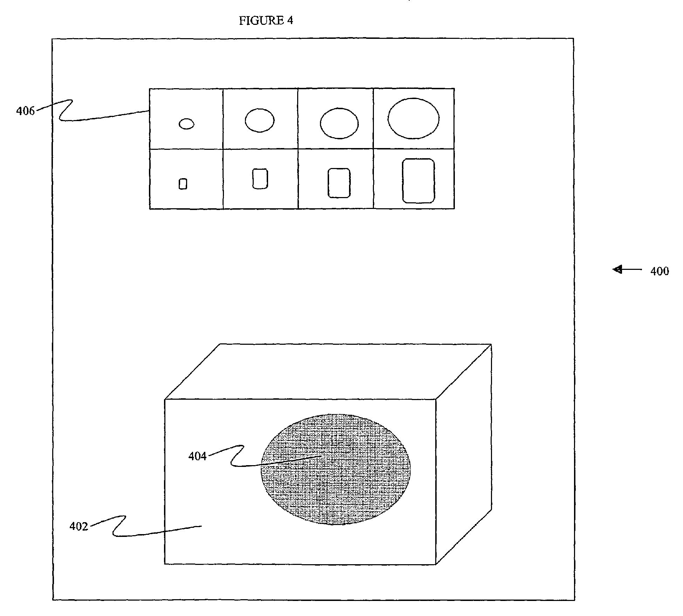

Turning for a moment to FIG. 4, an example of an application display 400 is shown displaying data and an interactive tool 406, in accordance with an embodiment of the present application. Interactive tool 406 selection may be provided through an icon, a menu, a floating menu, a contextual menu, and/or the like, for example. The interactive tool 406 may include a variety of tools, for example. The interactive tool 406 may include one or more tools selectable by a user, for example. The interactive tool 406 may have one or more tool tips for selection, for example. A tool tip may have a variety of sizes, shapes, diameters, and/or the like. A tool tip may impact a three-dimensional volume in a particular manner. The interactive tool 406 may also have other editable parameters, such as tool temperature, tool suction, duration of activation for a tool, and/or the like. In an embodiment, the interactive tool 406 is an ablation tool with a variety of tool tips. Each tool tip may impact surrounding anatomy in a differing way.

Turning back to FIG. 2, at step 206, an interaction is allowed between a user directing the interactive tool (such as tool 406) and the data set (such as the radiological image data 302 or 402 and/or a segmentation 304 or 404). A user may interact with the interactive tool and data set in two, three, and/or four dimensions, for example. A user may be able to position the interactive tool and/or activate the interactive tool, for example. If the interactive tool is an ablation tool, the user may be able to position the tool tip within radiological image data (e.g. 302/402) and/or a segmentation (e.g. 304/404), for example. Once in a satisfactory position, the user may then be able to activate an ablation tool, thereby causing a simulation that heat is provided through the tool tip, for example.

An application may be able to record the interaction of the user, and store in memory the interaction as part of a surgical planning analysis or surgical plan, for example. A user may be able to edit the surgical planning analysis or surgical plan by adding or removing, or otherwise altering interactions, for example. The surgical planning analysis or surgical plan may be storable in memory for subsequent use as a separate data set, or integrated and/or otherwise associated with the underlying radiological image and/or segmentation, for example.

For example, an interaction may be stored as a vector-based trajectory. A trajectory may help a user, such as an interventional radiologist, visualize an efficient path to insert an interactive tool, such as an ablation tool, while avoiding major anatomy. The trajectory may be displayed back to a user, for example, in real-time and/or otherwise.

At step 208, a prediction based on the user interaction is formed. A prediction may be based on the type of interactive tool (e.g. ablation tool), the type of tool tip for the interactive tool, the nature of the interaction, the heat of the interactive tool and/or tool tip, the position of the tool with respect to the region of anatomy, the angle of the tool with respect to the region of anatomy, the duration of tool activity, the type of anatomy in the region of interaction, and/or the like, for example. For example, an ablative tool may burn through certain types of anatomy more quickly and effectively than other types of anatomy. For example, larger tool tips for an ablative tool may burn through larger areas of anatomy. The application may be capable of recognizing some or all of these various factors, and predicting in response how the patient's anatomy will respond to the proposed interaction. Furthermore, the application may be capable of storing the prediction in memory. Further, a prediction may be displayable back to the user. Prediction feedback may be displayed to a user in real-time, for example. An application may record and store a prediction as part of a surgical planning analysis or surgical plan, for example, or as a separate data set, for example. A user may be able to edit the surgical planning analysis or surgical plan by adding or removing, or otherwise altering predictions, for example.

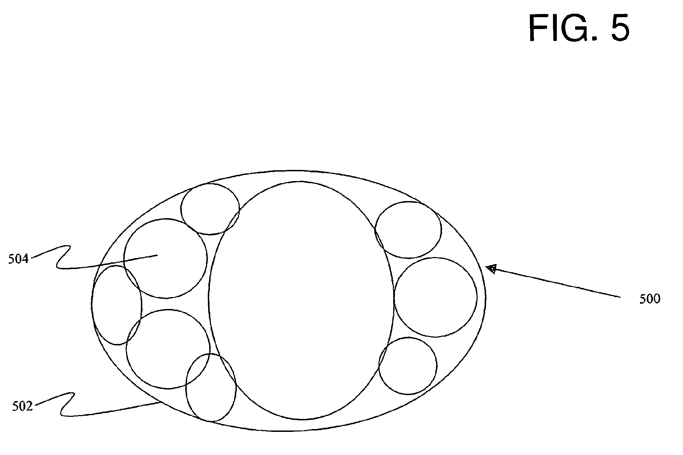

Turning for a moment to FIG. 5, an example of prediction forming 500 is shown, in accordance with an embodiment of the present invention. A segmentation 502 is shown. The segmentation 502 may be a segmentation of a tumor for example. Further, a number of varying predictions 504 based on user interactions are shown. Each prediction 504 may result from a user interaction with an interactive tool, such as an ablation tool. The interactive tool may have a variety of tool tips, for example, thus resulting in the variety of predictions 504, for example. The predictions 504 may be displayed to a user, and further stored as part of surgical planning analysis or surgical plan, for example.

Turning back to FIG. 2, as an illustrative example, method 200 may be performed in the following manner. A patient has a tumor which needs to be removed through ablation (e.g. thermal ablation or cryoablation). At step 202 an application displays data of a patient's anatomy including a tumor, and a corresponding segmentation of the tumor. A CT image of the patient was previously generated in three dimensions after the patient received a contrast agent. The image was transferred to a storage (such as storage 114), and was retrieved by a processor (such as processor 108) executing the application. The application was able to segment the tumor by an imaging protocol which filters non-tumor anatomy portions, based on corresponding intensity properties of the voxels. A shape was then fitted to the tumor tissue using an edge detection algorithm. The segmentation is displayed to the user through the application.

At step 204, the user is provided with an interactive ablation tool with a variety of tool tips through an interactive menu. The tool tips range in size and shape. A user may select one tool tip at a time. A user selects a tool tip through a user interface (such as user interface 110).

At step 206, the user interacts with the radiological image data and the segmentation of the tumor with the ablation tool and selected tool tip. The user interacts with the image and the segmentation through a user interface (such as user interface 110). As the user interacts with the data in the application, the interactions are recorded as part of a surgical planning analysis or surgical plan. As the ablation tool crosses through non-tumor anatomy to reach the tumor, the interaction is recorded. Once the ablation tool tip enters a region of the tumor, the user further interacts with the data by indicating that the ablation tool tip is to be heated. The user may indicate tool tip heating through, for example, clicking on a mousing device, for example.

At step 208, a prediction is formed based on the interaction at step 206. In this particular example, the application is designed to provide as much real-time feedback as possible to the user interacting with the data. Therefore, after every interaction, a prediction is made and displayed back to the user in real-time. Thus, each time the ablation tool crosses through non-tumor tissue while the tip is not hot, a resulting prediction of how the interaction impacts the non-tumor tissue is calculated (based on the size and shape of the tool tip, and the surrounding anatomy) and displayed back to the user in real-time. Each time the ablation tool is heated inside a region of the tumor, a prediction is calculated (based on tool tip size, tool tip shape, tool tip temperature, tool tip heating duration, and type of tumor) and displayed back to the user in real-time. The user may then edit the predictions as they are recorded by, for example, adding, deleting, and/or altering the predictions in accordance with clinical objectives. The set of predictions based on interactions is storable as a surgical planning analysis or surgical plan which may be retrieved at a later point in time, such as during or immediately before surgery, for example.

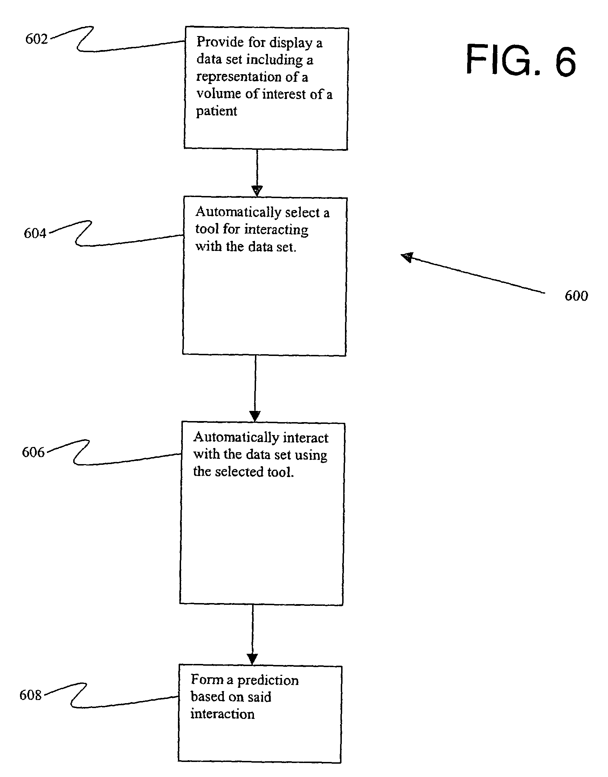

FIG. 6 shows a method 600 for performing automated surgical planning. The steps of method 600 may be performed in an alternate order as shown, for example. At least some of the steps of method 600 may be performed simultaneously in part, for example. Furthermore, some steps of method 600 may be omitted, for example. The steps of method may be performed by a computer and/or other processor (such as processor 108 in FIG. 1) executing a set of instructions on a computer-readable medium, for example. Further, some steps of method 200 may be interchanged with similar steps in method 600, and vice versa.

At step 602 a data set including a representation of a volume of interest of a patient is provided for display. Step 602 may be similar to step 202, for example.

At step 604, a tool for interacting with the data set is automatically selected. In many respects, step 604 may be similar to step 204. However, a tool may be automatically selected by an application, for example, based on a variety of factors. For example, if a tumor is to be removed through ablation (e.g. thermal ablation or cryoablation), an ablation tool tip size and shape may be selected to minimize the number of ablations needed to remove the tumor substantially. As another example, a tool tip size and shape may be selected automatically based on potential impact to non-tumor tissue along a projected entry path into the tumor tissue. A user may be able to override the automatic selection of an interactive tool, or may otherwise be able to tailor the rules used for automatic selection of the interactive tool, for example.

At step 606 the selected tool automatically interacts with the data set. In many respects, step 606 may be similar to step 206. However, a tool may automatically interact with data through an application, for example, based on a variety of factors. For example, if a tumor is to be removed through ablation (e.g. thermal ablation or cryoablation), an ablation tool may be guided through non-tissue anatomy along an efficient path into a particular region of the tumor. Once in position, the tool may be automatically actuated for a duration automatically calculated based on efficiency. A user may be able to override the automatic selection of an interactive tool, or may otherwise be able to tailor rules used for automatic selection of the interactive tool, for example. For example, a user may be able to constrain certain parameters such as tool tip size and/or tool tip temperature, while letting automated algorithm(s) determine other factors. Interactions may be stored in memory and/or saved as part of a surgical planning analysis or surgical plan. Interactions may be further edited by a user and/or an automatic planning algorithm by adding, removing, and/or altering interactions, for example.

At step 608 a prediction is formed based on the interaction. Step 608 may be similar to step 208, for example. Method 600 may automatically loop back to step 604 and/or 606 and continue until a particular automated planning and prediction process is complete, for example. Predictions may be stored in memory and/or saved as part of a surgical planning analysis or surgical plan. Predictions may be further edited by a user and/or an automatic planning algorithm by adding, removing, and/or altering predictions, for example.

As an illustrative example, method 600 may be performed in the following manner. A patient has a tumor that needs to be removed through ablation (e.g. thermal ablation or cryoablation). At step 602 an application displays data of a patient's anatomy including a tumor, and a corresponding segmentation of the tumor. A CT image of the patient was previously generated in three dimensions after the patient received a contrast agent. The image was transferred to a storage (such as storage 114), and was retrieved by a processor (such as processor 108) executing the application. The application was able to segment the tumor by an imaging protocol which filters non-tumor anatomy portions, based on corresponding intensity properties of the voxels. A shape was then fitted to the tumor tissue using an edge detection algorithm. The segmentation is displayed to the user through the application.

At step 604, the system automatically chooses an ablation tool tip that may efficiently remove the tumor, based on the perceived size of the tumor (e.g. the segmentation). The user is asked to confirm the choice of tool tips, and the user confirms the automatic selection of the interactive tool. For this example, the same size tool tip and shape will be used for all ablation tool interactions.

At step 606, the application automatically interacts with the radiological image data and the segmentation of the tumor with the ablation tool and selected tool tip. As the application interacts with the data, the interactions are recorded as part of a surgical planning analysis or surgical plan. As the ablation tool crosses through non-tumor anatomy to reach the tumor, the automatic interaction is recorded. Once the ablation tool tip enters a region of the tumor, the application further automatically interacts with the data by indicating that the ablation tool tip is to be heated for a specific duration and temperature.

At step 608, a prediction is formed based on the automatic interaction performed at step 606. For example, each time a tool moves through the image data, and each time the ablation tool is heated inside a region of the tumor, a prediction is calculated (based on tool tip size, tool tip shape, tool tip temperature, tool tip heating duration, and type of tumor). In this example, the display is not updated until the tumor has been substantially ablated, virtually. Therefore, the method 600 loops back to step 606 to perform further iterations until the tumor volume (e.g. segmentation) has been ablated. After all iterations have been performed, the display is updated to indicate all of the predictions that have been calculated and recorded. The user may then edit the predictions after they are recorded by, for example, adding, deleting, and/or altering the predictions in accordance with clinical objectives. For example, the user may perform subsequent iterations of method 200, for example. The set of predictions based on interactions is storable as a surgical planning analysis or surgical plan that may be retrieved at a later point in time, such as during or immediately before surgery, for example.

In an embodiment, an image processing subsystem 116 includes a computer-readable medium, such as a hard disk, floppy disk, CD, CD-ROM, DVD, compact storage, flash memory and/or other memory (such as memory 106). The medium may be in memory 106, processor 108, storage 114, and/or in a separate system. The medium may include a set of instructions capable of execution by a computer or other processor. The providing, display, interacting, selecting, automating, and predicting functions described above may be implemented as instructions on the computer-readable medium. For example, the set of instructions may include a provision routine that provides for display a data set including a representation of a volume of interest of a patient. Additionally, the set of instructions may include a provision routine that provides an interactive tool for use in conjunction with a data set. Additionally, the set of instructions may include an allowance routine that allows an interaction with the interactive tool and a portion of the data set. In an embodiment, an interaction is allowed with a user. In another embodiment, an interaction is allowed to proceed automatically. Additionally, the set of instructions may include a formation routine that forms a prediction for an effect on a portion of the data set based at least in part on the interaction. In an embodiment, the set of instructions may include a selection routine for selecting the interactive tool from a plurality of tool types. In an embodiment, the tool may be selected automatically.

Once the planning process is complete, a clinician may have a surgical plan including one or more trajectories and/or other surgical actions, such as ablations, for example. The surgical plan may be used during surgery, for example, to assist clinicians in performance of surgery. For example, a surgical plan may be displayed in an operating room for clinicians to view during surgery.

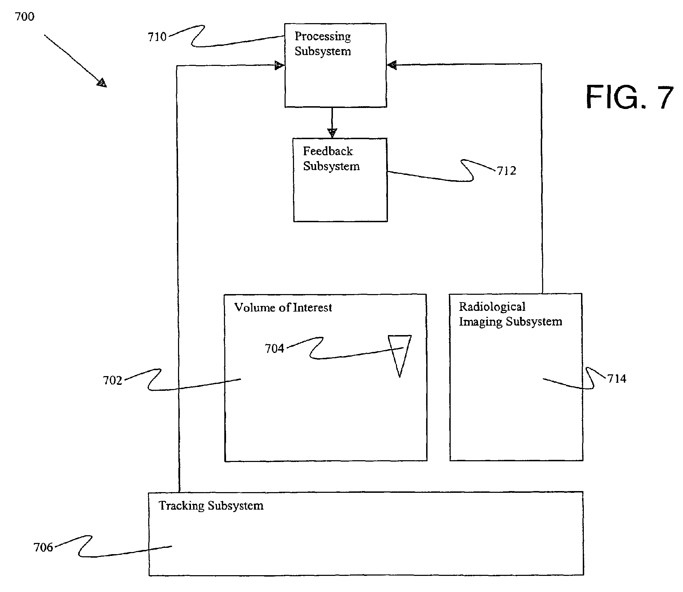

FIG. 7 shows a system 700 for facilitating surgery, in accordance with an embodiment of the present invention. A system 700 may include a volume of interest 702. A surgical implement 704 may be in the volume of interest. A position of the surgical implement may be tracked through a tracking subsystem 706. A radiological image of the volume of interest 702 (including a tool 704) may be generated with a radiological imaging subsystem 714. The tracking subsystem 706 and radiological subsystem 714 may output data to a processing subsystem 710, which may further control a feedback subsystem 712. One or more components, such as radiological imaging system 714, may be omitted from system 700, for example. One or more components may be integrated in various forms, or may be distributed across a plurality of components in various forms, for example.

A volume of interest 702 may include a volume of interest of a patient, for example. The volume of interest 702 may contain anatomy that is the focus of a surgical procedure, for example. A surgeon and/or an interventional radiologist, for example, may perform a surgical procedure using system 700. The volume of interest 702 may include other anatomy as well, for example. The volume of interest may correspond, at least in part, to the volume of interest used during surgical planning (e.g. methods 200 and/or 600). The volume of interest 704 may be oriented in a particular manner with respect to other components (e.g. tracking subsystem 706 and radiological imaging system 714) to improve surgical implement 704 tracking or for other purposes, for example.

A surgical implement 704 may be any implement for use in a volume of interest during a surgical procedure, for example. A surgical implement 704 may include an ablation tool, for example. The surgical implement 704 may be positioned in the volume of interest 702, for example. The surgical implement 704 may be positioned by a clinician (e.g. surgeon or interventional radiologist) or by automated means (e.g. automated scope, robotics, and/or the like). The surgical implement 704 may include a tip portion, for example. The tip portion may have a tip that performs a surgical action within the volume of interest 702, such as ablation (e.g. thermal ablation or cryoablation), for example. The tip portion may be interchangeable, for example. The surgical implement 704 may include an interaction portion, for example. The interaction portion may be any portion of a surgical implement 704 through which a clinician interacts with the surgical implement 794, for example. An interaction portion may include the handle of a surgical implement 704, or the control portion of a scope, for example. The surgical implement 704 may be integrated and/or in communication with a feedback subsystem 712, as will be further discussed. At least a portion (e.g. tip portion) of the surgical implement 704 may be tracked through a tracking subsystem 706, for example. It may be useful to include in a tool tip materials and/or devices that may facilitate tracking, such as particular metals and/or wireless/wired transmitters, for example. The entire surgical implement 704 may be trackable, or only a portion (e.g. tip) of the implement 704 may be tracked. The surgical implement 704, or a portion thereof, may correspond substantially to the interactive tool discussed in context of methods 200 and 600, for example.

A tracking subsystem 706 may include any subsystem capable of tracking a position of the surgical implement 704, for example. The tracking subsystem 706 may include an electromagnetic tracking subsystem, an optical tracking subsystem, a wireless receiver, a wired receiver, and/or the like. The tracking subsystem 706 may track a position of the surgical implement 704 in two, three, and/or four dimensions, for example. The tracking subsystem 706 may track the position of the implement 704 in real-time, for example. The tracking subsystem 706 may provide output for use with other systems, such as a processing subsystem 710 and/or a display, for example. The tracking subsystem 706 may be integrated with other systems, such as a radiological imaging subsystem 714. The tracking subsystem 706 may employ fiducials and/or markers to facilitate tracking and coordination of tracking data with other data, for example.

A radiological imaging subsystem 714 may generate and/or provide a radiological image of at least a portion of the volume of interest 702. The radiological imaging subsystem 714 may also generate and/or provide an image of the implement 704. The radiological imaging subsystem 714 may be integrated with a tracking subsystem 706, in whole or in part, for example. For example, a position of the implement 704 may be tracked in radiological image (e.g. in two, three, or four dimensions) through segmentation or other identification algorithms. The radiological imaging subsystem 714 may include CT, or ultrasound. For example, the radiological imaging subsystem 714 may include a four dimensional ultrasound imaging system capable of producing real-time images of the volume of interest during surgery. The radiological imaging subsystem 706 may employ fiducials and/or markers to facilitate imaging and coordination of imaging data with other data, for example.

A processing subsystem 710 may include an image processing subsystem, such as image processing subsystem 116 shown in FIG. 1, for example. The processing subsystem 710 may include a computer, processor, workstation, and/or the like, such as a PACS or Advantage® workstation, for example. The processing subsystem 710 may have a processor (such as processor 108) capable of executing an application from a set of instructions on a computer-readable medium, for example. The processing subsystem 710 may be capable of receiving data from tracking subsystem 706 and/or radiological imaging subsystem 714, for example. Further, the processing subsystem 710 may be capable of recognizing a surgical plan, such as a surgical plan generated in methods 200 and/or 600, for example. The processing subsystem 710 may be capable of uploading and/or receiving a previously generated surgical plan and storing it in memory (such as memory 106), for example. The processing subsystem 710 may have an application capable of recognizing the surgical plan and performing further processing tasks based on the plan, for example. The processing subsystem may be integratable in whole or in part with other components in system 700, such as radiological imaging subsystem 714 and/or tracking subsystem 706 for example.

The processing subsystem 710 may be capable of receiving and/or performing processing with at least four types of data: tracking subsystem 706 data, radiological imaging subsystem 714 data, previous radiological data, and/or surgical plan data, for example. Previous radiological data may be integrated with the surgical plan, for example. In particular, the tracking data and the surgical plan data may be coordinated, either automatically and/or through user intervention, for example. The tracking data and surgical plan data may be mapped, such that the position of the surgical implement 704 is mapped and/or correlated with positions in the surgical plan, for example. Furthermore, radiological imaging data, such as four dimensional ultrasound data may be further mapped and/or correlated with various of the other data sets to improve real-time imaging of a volume of interest during a surgical procedure, for example.

The various data (tracking, surgical plan, real-time radiological image) may be provided in a displayable form by the processing subsystem 710, for example. The data types may be overlapped, or otherwise indicated as being correlated or corresponding, for example. The data types may be integrated or may be displayed as separate frames, for example. The data types may be merged, or may be conceptually separable, for example.

The processing subsystem 710 may be able to compare the position of the implement 704 with a surgical plan and determine if the implement 704 is in the proper position, for example. Based on the correspondence between the tracked position of the implement 704 and the planned position and/or trajectory of a substantially similar implement 704 during surgical planning, the processing subsystem 710 may be able to control a feedback subsystem 712, for example. Other comparisons may also be possible including the following: surgical plan data versus radiological imaging subsystem 714 data, and tracking subsystem 706 data versus radiological imaging subsystem 714 data, for example. Any comparison that indicates the position of the implement 704 with respect to the expected position of the implement may result in the processing subsystem 710 controlling the feedback subsystem 712, for example. The manner and rules under which the processing subsystem 710 controls the feedback subsystem may be configurable, for example (e.g. type of feedback, duration of feedback, margin of error, etc.).

A feedback subsystem 712 may include any device(s) capable of communicating sensory information to a clinician and/or a device (e.g. robot) performing a procedure. A feedback subsystem 712 may be integrated in whole or in part into other portions of system 700, such as implement 704 and/or processing subsystem 710, for example. A feedback subsystem 712 may include haptic feedback or force feedback, capable of communicating sensory information to a clinician, for example. A haptic feedback may cause vibration(s) or otherwise produce motion to let a clinician know there is feedback. A type of feedback may be associated with an unplanned motion, for example. A vibration, auditory signal, optical signal, and/or the like may be communicated based on whether the implement 704 is on a planned trajectory and/or position, for example. A haptic feedback device may be incorporated into a portion of implement 704, such as the handle and/or control portion, for example. A clinician, such as one performing surgery, may receive feedback through ear(s), eye(s), and/or any portion of the body (e.g. foot), for example. Feedback subsystem 712 may communicate through wires, optical, infrared, or wireless connections, for example. The type of feedback may be configured based on clinician and/or design preferences. Various feedback may be provided under positive, negative, and/or neutral conditions, for example. An absence of signal may be a form of feedback, for example. Clinicians may prefer, for example, a design where "no news is good news," for example. Further, feedback may involve enabling and/or disabling surgical tools, such as the surgical implement, for example. The surgical implement may only function when positioned properly, for example.

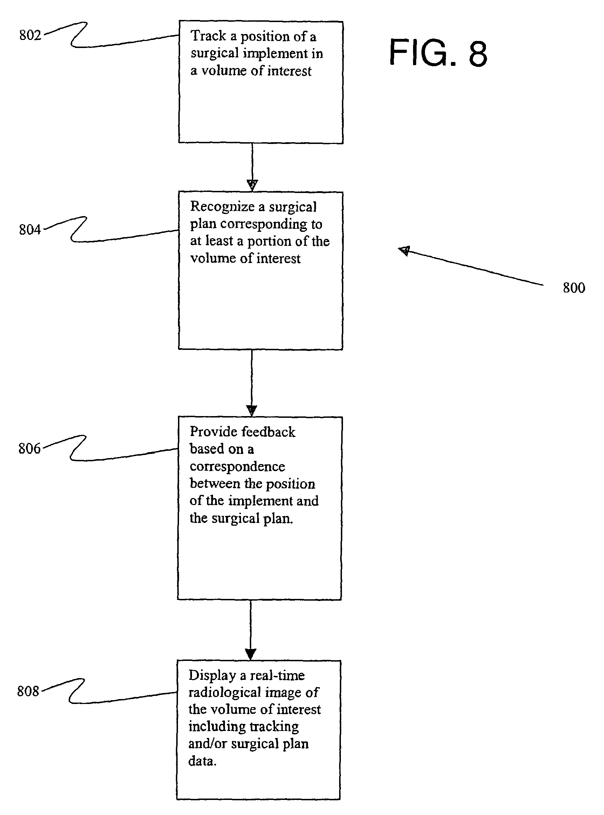

FIG. 8 shows a flowchart for a method 800 for facilitating surgery. The steps of method 800 may be performed in an alternate order as shown, for example. At least some of the steps of method 800 may be performed simultaneously or substantially simultaneously, for example. Furthermore, some steps of method 800 may be omitted, for example, such as step 808. The steps of method may be performed by a computer and/or other processor (such as processor 108 in FIG. 1) executing a set of instructions on a computer-readable medium, for example

At step 802, a position of a surgical implement (e.g. 704, shown in FIG. 7) in a volume of interest (e.g. 702) may be tracked. For example, a tracking subsystem (e.g. 706) may be employed to track a surgical implement. As another example, a radiological imaging subsystem (e.g. 714) may provide position information of an implement. Further, a combination of a plurality systems (e.g. tracking subsystem and radiological imaging subsystem) may be used together to provide position information for a surgical implement, for example. The position may correspond to an entire surgical implement, or only a portion thereof (e.g. a tip), for example. The position may be trackable in two, three, and/or four dimensions, for example. Tracking may employ fiducials and/or markers to facilitate data coordination and/or mapping, for example. A fiducial may indicate a reference location so that the tracking data may be contextually analyzed. Tracking may employ wireless, wired, optical, ultrasound, and/or other techniques, for example. For example, tracking may employ the receipt of electromagnetic signals from an implement to measure a position of the implement. Tracking data may be generated in real-time, and may be storable as discrete set(s) of data, and/or integrated with other data, for example.

At step 804, a surgical plan corresponding to at least a portion of the volume of interest (e.g. 702) may be recognized. For example, a surgical plan may be recognized by a processing subsystem (e.g. 710) capable of loading and/or uploading at least a portion of the surgical plan. The surgical plan may have been generated through methods, such as method 200 and/or 600, for example. The surgical plan may include trajectory information for a surgical implement and/or other actions, such as planned ablation positions (e.g. thermal or cryoablations). The surgical plan may also include information about a pathology, such as a segmentation of a tumor, for example. The surgical plan may include radiological image data, such as image data generated prior to surgery. The image data may be generated with a contrast agent in a volume of interest, for example. The plan may be two, three, and/or four dimensional, for example. The plan may be coordinated and/or mapped with other data types for use in conjunction with method 800, for example. The plan may be coordinated with position data tracked at step 802, for example.

At step 806, feedback may be provided based on a correspondence between the position of the implement and the surgical plan. For example, the position of the implement may not correspond to a planned trajectory for the implement. For example, the position of the implement may not correspond to a planned position for the implement. Further, the type of implement may not correspond to the implement in a relevant portion of the plan, for example. Based on the type of correspondence between the position and the plan, feedback may be provided, for example. Feedback may be provided through a feedback subsystem 712, for example. Feedback may be controlled through a processing subsystem 710, for example. An absence of signal may be a form of feedback, for example. Clinicians may prefer, for example, a design where "no news is good news," for example. Further, feedback may involve enabling and/or disabling surgical tools, such as the surgical implement, for example. The surgical implement may only function when positioned properly, for example.

Feedback may be capable of indicating position information to a clinician and/or automated device (e.g. robot), for example. Feedback may be haptic, auditory, optic, sensory, and/or the like, for example. For example, feedback may include vibrations transmitted to a clinician who is guiding the surgical implement. For example, positive feedback (e.g. any feedback information associated with an action and/or position corresponding to the surgical plan) may be indicated when there is a positive correlation between the plan and the action and/or position of the implement, for example. Negative feedback may be provided when there is a negative correlation between the plan and the action and/or position of the implement, for example. A clinician and/or automated device (e.g. robot) may be able to respond to the feedback information to take corrective action, for example. A clinician and/or automated device may continue a particular interaction without corrective action upon receiving positive feedback, for example. Feedback may be provided to the user in real-time, for example.

At step 808, a real-time radiological image of the volume of interest may be displayed, including tracking and/or surgical plan data., for example. A real-time radiological image may be generated by CT or ultrasound, for example. A real-time image (such as a four-dimensional ultrasound image) may be fused with a prior radiological image (such as a three-dimensional reconstruction image), for example. Contrast agent may not be employed during surgery, so fusion of a prior image using contrast with a real-time image without contrast may enhance certain features of the display, such as the ability to recognize pathologies and other anatomy, for example. Various data types may be fused into a single image, or may be displayed as separate panes, or may be integrated in various forms. A user may be able to interact with the images, for example (e.g. rotation, pixel adjustment, etc.). The surgical plan data may include trajectories and positioning data, for example, and may be displayed in context with a real-time image of the volume of interest, for example. Fiducials and/or markers may be employed to correlate the various data types for display. A user may be able to select various views of the displayed data (e.g. axial, coronal, sagittal, oblique, etc.). Feedback information may be incorporated in the display, for example. A color of the tracked implement may change based on whether the implement is conforming to the surgical plan, for example.

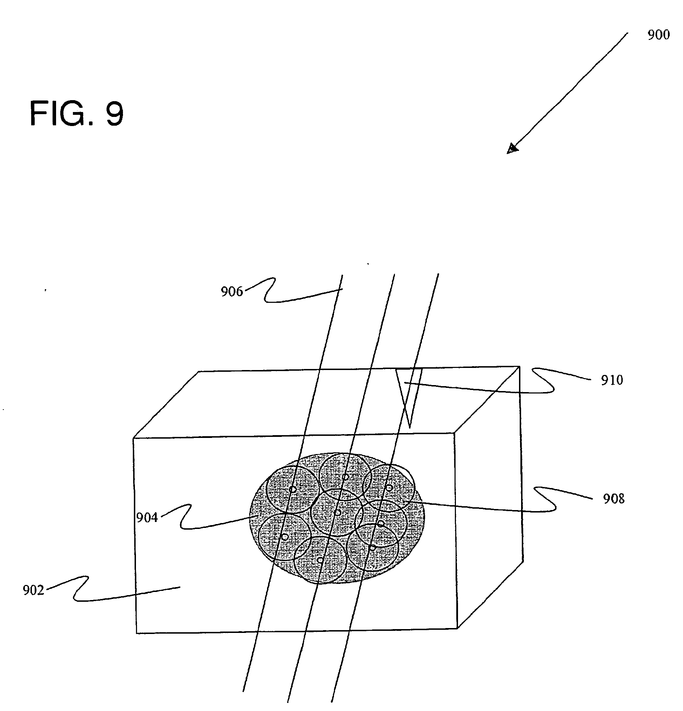

FIG. 9 shows an example 900 of a combination display including a surgical plan and a three-dimensional real-time image of a volume of interest, in accordance with an embodiment of the present invention. A volume of interest 902 may include real-time ultrasonic data fused with a prior three-dimensional CT scan (taken with contrast in the patient), for example. A prior three-dimensional CT scan may be a three-dimensional reconstruction scan. In the volume of interest 902 a segmented structure 904, such as a segmented tumor, may be included, for example. Segmentation may have been performed during prior imaging and/or processing, for example, in accordance with method 200 and/or 600, for example. A surgical plan, including trajectories 906 and positions 908 may be mapped onto the real-time image, for example. The surgical plan may be mapped onto the image through Boolean union or the like. The positions 908 include a tip position (which may be the smaller ring, and the predicted result of ablation, when the tool is actuated with the tip at the tip position, for example. The display also includes a surgical implement 910, such as a tip of an ablation tool, for example.

An illustrative example of method 800 may be performed as follows. An interventional radiologist is performing a surgical ablation (either thermal or cryoablation) of a tumor in the patient's volume of interest. The radiologist guides an ablation tool to perform the ablation. At step 802, an electromagnetic tracking subsystem tracks the position of an ablation tool as it is guided by the radiologist in a patient's volume of interest.. The system tracks the position of the tool tip in four dimensions (three dimensions over time). Further, the system tracks the tool substantially in real-time. At step 804, a processing subsystem has recognized a surgical plan generated previously, in accordance with methods 200 and 600. The plan has trajectories by which the ablation tool is supposed to follow. Further, the plan has locations at which the ablation tool should be activated to burn and/or freeze the tumor tissue. The plan also has a previously generated three-dimensional radiological image of the patient's volume of interest. The previously generated image was generated by CT scan with the assistance of a contrast agent in the volume of interest. The processing subsystem is designed to recognize the surgical plan, and to perform processing on the plan.

At step 806, the processing subsystem compares the position of the implement with the plan. If the position and trajectory of the ablation tool corresponds to the surgical plan, then the tool functions properly, and no signal is provided. The absence of a signal indicates to the clinician that the tool is positioned and is functioning as planned. If, however, the radiologist deviates from the surgical plan, a vibration is provided to the radiologist to indicate that the plan is not being followed. The vibration is a subtle vibration at the point where the radiologist's hand meets the ablation tool. The vibration is sufficient to alert the radiologist, but is not strong enough to otherwise move the ablation tool. A radiologist may choose to override the plan based on real-time circumstances (e.g. an emergency), or may choose to take corrective action based on the feedback (e.g. reposition the tool to correspond more substantially to the surgical plan).

At step 808, a real-time ultrasonic four dimensional image is displayed to the surgeon. The ultrasonic image has been fused with a three dimensional image of the same volume generated prior to surgery with a CT scan. The prior image was generated with a contrast agent. The fusion of the two images produces enhanced anatomical visibility in the display. Further, the surgical plan, having the trajectories and ablation tool positions is displayed. In addition, the current position of the ablation tool is also displayed. In this particular example, all data is displayed together in a single three-dimensional image in real time. However, the application also allows various two-dimensional views to be displayed simultaneously, including axial, coronal, sagittal, and/or oblique views.

Turning to FIG. 7, in an embodiment, a processing subsystem 710 and/or a tracking subsystem 706 include a computer-readable medium, such as a hard disk, floppy disk, CD, CD-ROM, DVD, compact storage, flash memory and/or other memory (such as memory 106). The medium may be in memory 106, processor 108, storage 114, and/or in a separate system. The medium may include a set of instructions capable of execution by a computer or other processor. The tracking, feedback, and processing functions described above may be implemented as instructions on the computer-readable medium. For example, the set of instructions may include a tracking routine for tracking a position of at least a portion of a surgical implement in a volume of interest. Additionally, the set of instructions may include a recognition routine provision routine for recognizing a surgical plan corresponding to at least a portion of the volume of interest. Additionally, the set of instructions may include a feedback routine for providing feedback based on a correspondence between said position of said at least a portion of said surgical implement and said surgical plan. In an embodiment, feedback is provided in real-time. In another embodiment, Additionally, the set of instructions may include a display routine for displaying a real-time radiological image of at least a portion of the volume of interest, wherein the real-time radiological image corresponds to the surgical plan.

Thus, embodiments of the present application provide systems that reduce risks associated with surgical procedures. Additionally, embodiments of the present application provide systems that automatically provide pre-operative plans for later use in a surgical setting. Moreover, embodiments of the present application provide systems that assist clinicians in following pre-operative plans during a surgery.

While the invention has been described with reference to certain embodiments, it will be understood by those skilled in the art that various changes may be made and equivalents may be substituted without departing from the scope of the invention. In addition, many modifications may be made to adapt a particular situation or material to the teachings of the invention without departing from its scope. For example, features may be implemented with software, hardware, or a mix thereof. Therefore, it is intended that the invention not be limited to the particular embodiment disclosed, but that the invention will include all embodiments falling within the scope of the appended claims.

## Claims

1. A system (100, 700) for facilitating surgery comprising:
a tracking subsystem (706) for tracking a position of at least a portion of a surgical implement (704, 910) in a patient;
a feedback subsystem (712) capable of providing a feedback response; and
an application executable, at least in part, on a processor (108, 710), said application being capable of comparing said position of said at least a portion of said surgical implement (704, 910) and a surgical plan (906, 908), said application being capable of controlling said feedback subsystem (712) in response to a correlation between said position of said at least a portion of said surgical implement (704, 910) and said surgical plan (906, 908); and **characterized in that**
the system further comprises :
means for providing for display a data set including a representation of a volume of interest (702) of a patient;
an interactive tool (406) representative of said at least a portion of a surgical implement (704, 910) for use in conjunction with the data set;
means for allowing an interaction between a user directing said interactive tool and said data set;
means for forming a prediction (504) how patient's anatomy will respond to the proposed interaction based on said interaction;
means for recording said interaction of the user; and
means for storing said recorded interaction of the user as a part of a surgical plan (906, 908).

2. The system (100, 700) of claim 1, wherein said surgical plan (906, 908) comprises at least one trajectory (906) and at least one position.

3. The system (100, 700) of claim 1, wherein said feedback response comprises at least one of: haptic feedback, thermal feedback, optical feedback, and auditory feedback.

4. The system (100, 700) of claim 1, wherein said feedback response is provided in real-time.

5. The system (100, 700) of claim 1 further comprising a displayable output generated by said application, wherein said displayable output comprises a real-time radiological image (902) of at least a portion of said patient, wherein said real-time radiological image (902) corresponds to said surgical plan (906, 908).

## Patentansprüche

1. System zur Erleichterung chirurgischer Eingriffe, aufweisend:
ein Verfolgungs-Subsystem (706) zum Verfolgen einer Position wenigstens eines Abschnittes eines chirurgischen Instruments (704, 910) in einem Patienten;
ein Rückmeldungs-Subsystem (712), das eine Rückmeldungsantwort liefern kann; und
ein Anwendungsprogramm, das wenigstens teilweise auf einem Prozessor (108, 710) ausgeführt werden kann, wobei das Anwendungsprogramm die Position des wenigstens einen Abschnittes des chirurgischen Instruments (704, 910) und einen chirurgischen Plan (906, 908) vergleichen kann, wobei das Anwendungsprogramm das Rückmeldungs-Subsystem (712) in Reaktion auf eine Korrelation zwischen der Position des wenigstens einen Abschnittes des chirurgischen Instruments (704, 910) und des chirurgischen Plans (906, 908) steuern kann; und
**dadurch gekennzeichnet, dass**
das System ferner aufweist:
eine Einrichtung, um einen eine Repräsentation eines interessierenden Volumen (702) eines Patienten enthaltenden Datensatz zum Anzeigen zu liefern;
ein wenigstens einen Abschnitt eines chirurgischen Instruments (704, 910) repräsentierendes interaktives Werkzeug (406 zur Verwendung in Verbindung mit dem Datensatz;
eine Einrichtung zum Ermöglichen einer Wechselwirkung zwischen einem das interaktive Werkzeug führenden Anwender und dem Datensatz;
eine Einrichtung, um auf der Basis der Wechselwirkung eine Vorhersage (504) zu erzeugen, wie die Anatomie des Patienten auf die vorgeschlagene Wechselwirkung reagieren wird;
eine Einrichtung zum Aufzeichnen der Wechselwirkung mit dem Anwender; und
eine Einrichtung zum Speichern der aufgezeichneten Wechselwirkung mit dem Anwender als Teil eines Plans eines chirurgischen Eingriffs (906, 908).

2. System nach (100, 700) nach Anspruch 1, wobei der Plan des chirurgischen Eingriffs (906, 908) wenigstens eine Bewegungsbahn (906) und wenigsten eine Position enthält.

3. System nach (100, 700) nach Anspruch 1, wobei die Rückmeldungsantwort wenigstens eine aufweist, von: haptischer Rückmeldung, thermischer Rückmeldung, optischer Rückmeldung und akustischer Rückmeldung.

4. System nach (100, 700) nach Anspruch 1, wobei die Rückmeldungsantwort in Echtzeit geliefert wird.

5. System nach (100, 700) nach Anspruch 1, das ferner ein von dem Anwendungsprogramm erzeugtes anzeigbares Ergebnis aufweist, wobei das anzeigbare Ergebnis ein radiologisches Echtzeitbild (902) von wenigstens einem Abschnitt eines Patienten aufweist, wobei das radiologisches Echtzeitbild (902) dem Plan des chirurgischen Eingriffs (906, 908) entspricht.

## Revendications

1. Système (100, 700) pour faciliter la chirurgie comprenant :
un sous-système de pistage (706) pour pister une position d'au moins une partie d'un instrument chirurgical (704, 910) dans un patient ;
un sous-système de rétroaction (712) pouvant fournir une réponse rétroactive ; et
une application exécutable, au moins en partie, sur un processeur (108, 170), ladite application pouvant comparer ladite position de ladite au moins une partie dudit instrument chirurgical (704, 910) et un plan chirurgical (906, 908), ladite application pouvant commander ledit sous-système de rétroaction (712), en réponse à une corrélation entre ladite position de ladite au moins une partie dudit instrument chirurgical (704, 910) et ledit plan chirurgical (906, 908) ; et **caractérisé en ce que**
le système comprend en outre :
des moyens pour permettre l'affichage d'une série de données incluant une représentation d'un volume d'intérêt (702) d'un patient ;
un outil interactif (406) représentatif de ladite au moins une partie d'un instrument chirurgical (704, 910) pour utiliser en conjonction avec la série de données ;
des moyens pour permettre une interaction entre un utilisateur dirigeant ledit outil interactif et ladite série de données ;
des moyens pour former une prévision (504) de comment l'anatomie du patient répondra à l'interaction proposée en fonction de ladite interaction ;
des moyens pour enregistrer ladite interaction de l'utilisateur ; et
des moyens pour stocker ladite interaction enregistrée de l'utilisateur comme une partie d'un plan chirurgical (906, 908).

2. Système (100, 700) selon la revendication 1, dans lequel ledit plan chirurgical (906, 908) comprend au moins une trajectoire (906) et au moins une position.

3. Système (100, 700) selon la revendication 1, dans lequel ladite réponse rétroactive comprend au moins l'une parmi : la rétroaction haptique, la rétroaction thermique, la rétroaction optique, et la rétroaction auditive.

4. Système (100, 700) selon la revendication 1, dans lequel ladite réponse rétroactive est fournie en temps réel.

5. Système (100, 700) selon la revendication 1, comprenant en outre une sortie affichable générée par ladite application, dans lequel ladite sortie affichable comprend une image radiologique en temps réel (902) d'au moins une partie dudit patient, dans lequel ladite image radiologique en temps réel (902) correspond au dit plan chirurgical (906, 908).
